Europäisches Patentamt

(19) European Patent Office

Office européen des brevets

(11) EP 1 139 789 B1

## (12) EUROPÄISCHE PATENTSCHRIFT

(45) Veröffentlichungstag und Bekanntmachung des Hinweises auf die Patenterteilung:
**18.08.2004 Patentblatt 2004/34**

(51) Int Cl.[7]: **A23L 1/09**, C08L 3/00, A61K 9/20, A61K 31/715, A23K 1/16

(21) Anmeldenummer: **99973532.7**

(22) Anmeldetag: **30.11.1999**

(86) Internationale Anmeldenummer:
**PCT/EP1999/009298**

(87) Internationale Veröffentlichungsnummer:
**WO 2000/038537 (06.07.2000 Gazette 2000/27)**

(54) **ALPHA-AMYLASE-RESISTENTE STÄRKE ZUR HERSTELLUNG VON NAHRUNGS- UND ARZNEIMITTELN**

ALPHA-AMYLASE-RESISTANT STARCH FOR PRODUCING FOODSTUFF AND MEDICAMENTS

AMIDON ALPHA-AMYLASE RESISTANT UTILISE POUR PREPARER DES PRODUITS ALIMENTAIRES ET DES MEDICAMENTS

(84) Benannte Vertragsstaaten:
**AT BE CH CY DE DK ES FI FR GB GR IE IT LI LU MC NL PT SE**

(30) Priorität: **28.12.1998 DE 19860375**

(43) Veröffentlichungstag der Anmeldung:
**10.10.2001 Patentblatt 2001/41**

(73) Patentinhaber: **Südzucker Aktiengesellschaft Mannheim/Ochsenfurt**
**76283 Mannheim (DE)**

(72) Erfinder:
- **BENGS, Holger**
  **D-60598 Frankfurt (DE)**
- **BRUNNER, Anette**
  **D-91154 Roth (DE)**

(74) Vertreter: **Luderschmidt, Schüler & Partner Patentanwälte**
**Industriepark Höchst**
**65926 Frankfurt (DE)**

(56) Entgegenhaltungen:
**EP-A- 0 506 166     EP-A- 0 688 872**
**EP-A- 0 846 704     WO-A-97/34932**
**DE-A- 19 830 618**

- **PATENT ABSTRACTS OF JAPAN vol. 1996, no. 04, 30. April 1996 (1996-04-30) & JP 07 313069 A (SAPPORO BREWERIES LTD), 5. Dezember 1995 (1995-12-05)**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft Zusammensetzungen, die $\alpha$-1,4-D-Glucane und/oder eine resistente Stärke daraus enthalten, zur Herstellung von Nahrungsmitteln. Die Erfindung betrifft ferner die Verwendung resistenter Stärke als Arzneimittel.

Ernährungsstudien haben gezeigt, daß falsche und einseitige Ernährung Grund zahlreicher Erkrankungen, beispielsweise von Darmerkrankungen und insbesondere von Kolonkarzinomen sind. Epidemiologische Untersuchungen zeigen, daß bei einer fettreichen und ballaststoffarmen Ernährung ein erhöhtes Risiko für die Entstehung entzündlicher Darmerkrankungen und Kolonkrebs beim Menschen besteht. Ballaststoffreicher Nahrung schreibt man dagegen einen protektiven Effekt gegen kolorektale Erkrankungen zu.

Die Herstellung von sog. "Functional Food", d.h. Lebensmitteln, die nicht nur der Ernährung dienen sondern auch die Gesundheit fördern sollen, hat daher in letzter Zeit zunehmend an Bedeutung gewonnen. Solche Lebensmittel sind mit Zusätzen mit gesundheitsfördernder Wirkung angereichert.

Einer der Zusätze, der für die Lebensmittelindustrie zunehmend Bedeutung erlangt hat, ist resistente Stärke (RS), d.h. Stärke, die durch $\alpha$-Amylasen nicht abgebaut wird. Resistente Stärke wird demnach im Dünndarm des gesunden Menschen nicht verdaut und gelangt somit in den Dickdarm. Resistente Stärken in Lebensmitteln stellen somit eine energiereduzierte, körpergebende Komponente im Sinne eines Ballaststoffs dar.

Mit der Aufnahme RS-haltiger Lebensmittel sind jedoch auch noch zwei weitere Funktionen verknüpft, nämlich die Substratbereitstellung für den Energiestoffwechsel der intestinalen Mikroflora und den der Dickdarmepithelzellen. Resistente Stärke wird durch die intestinalen Mikroorganismen oxidativ zu kurzkettigen Fettsäuren wie Acetat, Propionat und Butyrat abgebaut. Diese kurzkettigen Fettsäuren wiederum dienen den Dickdarmepithelzellen, die insbesondere auf die luminale Zufuhr von Butyrat angewiesen sind, zur Aufrechterhaltung ihrer Struktur und Funktion.

Hohe luminale Butyratspiegel im Kolon können außerdem einen Schutzfaktor gegen kolorektale Erkrankungen darstellen. Während Butyrat nämlich in normalen Kolonozyten das Wachstum über eine Kette von Reaktionen steuert, die in normalen Kolonepithelzellen die Proliferation erhöht, scheint es die neoplastische Entwicklung von Kolonozyten zu unterdrücken.

Resistente Stärken kommen in der Natur nur in geringer Menge vor und entstehen während der Rekristallisation (Retrogradation) verkleisterter Stärke. Dabei bilden sich mikrokristalline Filamente, die ein Netzwerk ausbilden, wodurch eine enzymatische Hydrolyse verhindert wird.

Resistente Stärken, Verfahren zu ihrer Herstellung und ihre Verwendung in Lebensmitteln sind seit langem bekannt.

So beschreibt die US-A-3 729 380 die gezielte enzymatische Behandlung von Stärke, um den Gehalt an hochverzweigtem Amylopektin zu reduzieren und den Anteil an kurzkettigen Amylosestrukturen, die gewöhnlich eine stärkere Tendenz zur Retrogradation und damit zur Bildung resistenter Stärke als native Stärken besitzen, zu erhöhen.

Die EP-A-0 564 893 beschreibt ein Verfahren zur Herstellung von RS-Produkten, bei dem eine wässrige Suspension einer Stärke verkleistert und mit einem Enzym, das die $\alpha$-1,6-glykosidischen Bindungen spaltet, entzweigt wird. Das entstandene Zwischenprodukt wird anschließend retrogradiert.

Die EP-A-0 688 872 beschreibt ein Verfahren zur Herstellung von RS-Produkten, bei dem eine wässrige Suspension einer partiell abgebauten, verkleisterten Stärke enzymatisch entzweigt und das Zwischenprodukt retrogradiert wird.

In der US-A-5 776 887 wird eine Lebensmittelzusammensetzung für Diabetiker mit unterschiedlichen Kohlenhydratfraktionen offenbart, die unterschiedlich schnell absorbiert werden. Die langsam absorbierte Fraktion besteht dabei aus einer Maisstärke, die auch einen Gehalt an resistenter Stärke aufweist. Die US-A-5 776 887 stellt sich die Aufgabe, Kohlenhydrate über einen längeren Zeitraum gleichmäßig freizusetzen, um überhöhte Glucosespiegel zu vermeiden.

Die EP-A-0846704 offenbart nahezu völlig entzweigte Stärke zur Verwendung in Nahrungs- oder Futtermitteln.

Es besteht jedoch weiterhin ein starker Bedarf an verbesserten Nahrungsmitteln und Präparaten, die durch Zufuhr gesundheitsfördernder Stoffe das Wohlempfinden des Konsumenten steigern, einer fehlerhaften Ernährung vorbeugen können und Krankheiten, beispielsweise ernährungsbedingter Natur, heilen helfen.

Aufgabe der vorliegenden Erfindung war es daher, Lebens- und Futtermittel (im folgenden kollektiv Nahrungsmittel genannt) einerseits sowie pharmazeutische und veterinärmedizinische Mittel (im folgenden kollektiv Arzneimittel genannt) andererseits bereitzustellen, die den Erhalt der Gesundheit fördern, das Wohlbefinden stärken und zur Behandlung und/oder Vorbeugung von Erkrankungen, beispielsweise infolge von Mangelernährung, bei Mensch oder Tier eingesetzt werden können.

Diese Aufgabe wurde durch den Einsatz von wasserunlöslichen linearen $\alpha$-1,4-D-Glucanen in Kombination mit Lebens- oder Futtermitteladditiven zur Herstellung der oben genannten Produkte gelöst. In diesem Sinne versteht sich die vorliegende Erfindung als Weiterentwicklung der älteren, nicht vorveröffentlichten DE-A-198 30 618, die die Verwendung wasserunlöslicher α-1-4-D-Glucane zur Herstellung resistenter Stärke beschreibt.

Gegenstand der vorliegenden Erfindung sind demnach Zusammensetzungen, insbesondere zur Nahrungsergänzung, die ein wasserunlösliches lineares α-1,4-D-Glucan und/oder eine daraus erhältliche resistente Stärke, wobei linear bedeutet, daß der Verzweigungsgrad in 2-, 3- oder 6-Position den Wert 0,5% nicht überschreitet, und wenigstens ein weiteres Lebens- oder Futtermitteladditiv enthalten, ausgewählt aus der Gruppe bestehend aus Probiotika, Prebiotika, Vitaminen und Provitaminen, Antioxidantien, Ölen, Fetten und Fettsäuren, sowie Mischungen daraus.

Gegenstand der Erfindung sind ferner Nahrungsmittel, die mit diesen Zusammensetzungen erhältlich sind.

Gegenstand der vorliegenden Erfindung ist ferner die Verwendung resistenter Stärke auf Basis wasserunlöslicher linearer α-1,4-D-Glucane als Ersatzstoff und/oder Kalorienreduktionsmittel in Nahrungsmitteln.

Gegenstand der vorliegenden Erfindung sind schließlich die Verwendung resistente Stärken auf Basis wasserunlöslicher linearer α-1,4-D-Glucane als Arzneimittel und pharmazeutische oder veterinärmedizinische Zusammensetzungen, die solche resistenten Stärken enthalten.

Es wurde überraschend gefunden, daß wasserunlösliche lineare α-1,4-D-Glucane bei der Retrogradation in großer Menge hochresistente Stärke, insbesondere vom Typ RS-III, liefern (Englyst et al. (Classification and measurement of nutritionally important starch Fractions, European Journal of Clinical Nutrition, 46 (Suppl. 23) (1992) 33-50).

Dies macht diese resistenten Stärken als Ersatzstoff für Nahrungsmittelinhaltsstoffe, insbesondere als Ballaststoffersatz und als Fettstoffersatz, und damit auch als Kalorienreduktionsmittel geeignet.

Es wurde ferner gefunden, daß die aus wasserunlöslichen linearen α-1,4-D-Glucanen erhältliche resistente Stärke beim Abbau im Dickdarm nicht nur zu einer großen Menge kurzkettiger Fettsäuren, sondern insbesondere auch zu einem hohen Anteil der besonders vorteilhaften Butyrate führt.

Es wurde ferner gefunden, daß die aus wasserunlöslichen linearen α-1,4-D-Glucanen erhältliche resistente Stärke in Kombination mit anderen Lebens- oder Futtermitteladditiven einen synergistischen oder symbiotischen Effekt zeigt, indem sich die Wirkungen der Komponenten gegenseitig verstärken.

Unter wasserunlöslichen linearen α-1,4-D-Glucanen werden im folgenden die nicht α-Amylase-resistenten Formen dieser Glucane verstanden.

Unter wasserunlöslichen α-1,4-D-Glucanen werden hierbei α-1,4-D-Glucane verstanden, die nach der Definition des Deutschen Arzneimittelbuches (DAB, Wissenschaftliche Verlagsgesellschaft mbH, Stuttgart, Gori-Verlag GmbH, Frankfurt, 9. Auflage, 1987) entsprechend den Klassen 4-7 unter die Kategorien "wenig löslich", "schwer löslich", "sehr schwer löslich" und "praktisch unlöslich" fallen.

Die Wasserunlöslichkeit der erfindungsgemäß eingesetzten α-1,4-D-Glucane ist zweckmäßig derart, daß wenigstens 98%, insbesondere wenigstens 99,5%, der eingesetzten Polysaccharide unter Normalbedingungen (T = 25°C +/- 20%; p = 101325 Pascal +/- 20%) in Wasser unlöslich sind (entsprechend mindestens den Klassen 4 und 5 nach DAB).

Vorteilhaft entspricht die Wasserunlöslichkeit der eingesetzten a-1,4-D-Glucane den Klassen 6 oder 7 nach DAB.

Unter linearen α-1,4-D-Glucanen werden α-1,4-D-Glucane verstanden, deren Verzweigungsgrad maximal 4% beträgt, d.h. deren Hauptkette maximal vier Seitenketten auf 100 Monosaccharideinheiten aufweist.

Besonders bevorzugt sind wasserunlösliche lineare α-1,4-D-Glucane, die keine Verzweigungen aufweisen bzw. deren Verzweigungsgrad so minimal ist, daß er mit herkömmlichen Methoden nicht mehr nachweisbar ist.

Die gewichtsgemittelten Molekulargewichte $M_w$ (bestimmt mittels Gelpermeationschromatographie im Vergleich zu einer Eichung mit Pullulanstandard) der erfindungsgemäß verwendeten wasserunlöslichen linearen α-1,4-D-Glucane können in einem weiten Bereich variieren. Zweckmäßig besitzen die als Ausgangsmaterial eingesetzten wasserunlöslichen linearen α-1,4-D-Glukane ein Molekulargewicht $M_w$ von 0,75 x $10^2$ bis $10^7$ g/mol, bevorzugt von $10^3$ bis $10^6$ g/mol und besonders bevorzugt von $10^3$ bis $10^5$ g/mol. Ein ganz besonders vorteilhafter Bereich liegt zwischen 2 x $10^3$ g/mol und 8 x $10^3$ g/mol.

Besonders bevorzugt weisen die verwendeten wasserunlöslichen linearen α-1,4-D-Glucane einen hohen Anteil an Polymeren mit einem Polmerisationsgrad zwischen 10 und 35 auf. Vorteilhaft weisen wenigstens 25%, bevorzugt wenigstens 30% und besonders bevorzugt wenigstens 35% der eingesetzten α-1,4-D-Glucanmoleküle einen Polymerisationsgrad von 10 bis 35 auf.

Die Polydispersität $M_w/M_n$ der verwendeten wasserunlöslichen linearen α-1,4-D-Glucane kann innerhalb weiter Bereiche variieren. Bevorzugte Werte für die Polydispersität liegen im Bereich von 1,01 bis 50, insbesondere von 1,5 bis 15. Die Verwendung von Glucanen mit niedrigerer Polydispersität ist aber wegen der besseren Reproduzierbarkeit der erhaltenen Produkte bevorzugt.

Die verwendeten α-1,4-D-Glukane können auch chemisch in an sich bekannter Weise modifiziert sein, solange die Modifikationen im Hinblick auf die herzustellenden Nahrungs- und Arzneimittel unbedenklich sind. So können die α-1,4-D-Glukane durch Veretherung oder Veresterung in 2-, 3- oder 6-Position in dem Fachmann bekannter Weise chemisch modifiziert sein. (s.a. Functional Properties of Food Components, 2nd edition, Y. Pomeranz, Academic Press (1991); Lehrbuch der Lebensmittelchemie, Belitz & Grosch, Springer Verlag (1992); Citrat Starch Possible Application as Resistent Starch in Different Food Systems, B. Wepner et al., European Air Concerted Action, Abstract: air3ct94-2203, Functional Properties of Non-digestible Carbohydrates, Pro Fibre-Tagung, Lissabon, Februar 1998, Seite 59). Bevorzugt werden jedoch chemisch unmodifizierte α-1,4-D-Glucane verwendet.

Die erfindungsgemäß verwendeten wasserunlöslichen linearen α-1,4-D-Glucane können beliebigen Ursprungs sein.

Beispielsweise können die wasserunlöslichen linearen α-1,4-D-Glucane aus natürlichen pflanzlichen und tierischen Quellen oder aus Mikroorganismen, die solche Glucane produzieren, durch konventionelle Isolierung und Aufreinigung erhalten werden.

Da die meisten natürlichen Quellen die gewünschten wasserunlöslichen linearen α-1,4-D-Glucane aber nicht in den gewünschten Mengen enthalten, werden diese Polysaccharide vorteilhaft auf biotechnischem Wege gewonnen. Beispielsweise können die natürlichen Produzenten der wasserunlöslichen linearen Glucane gentechnisch derart manipuliert werden, daß sie im Vergleich mit dem unmanipulierten Organismus einen höheren Anteil an nicht oder nur geringfügig verzweigten Polysacchariden enthalten.

Die gewünschten wasserunlöslichen linearen α-1,4-D-Glucane können auch aus hochverzweigten Glucanen durch chemische oder enzymatische Entzweigung, beispielsweise mit Entzweigungsenzymen wie Pullulanasen erhalten werden.

Bevorzugt werden die erfindungsgemäß eingesetzten α-1,4-D-Glucane durch Biotransformation oder biokatalytisch hergestellt.

Unter Biotransformation oder biokatalytischer Herstellung wird hier verstanden, daß das wasserunlösliche α-1,4-D-Glucan in vitro durch katalytische Polymerisation von Glucosemolekülen unter Einwirkung eines geeigneten Enzyms, insbesondere eines Enzyms mit Amylosucrase-Aktivität, unter geeigneten Bedingungen hergestellt wird.

Ein vorteilhaftes biokatalytisches Verfahren zur Gewinnung wasserunlöslicher linearer α-1,4-D-Glucane wird in der WO 95/31553 beschrieben, deren Offenbarungsgehalt ausdrücklich Gegenstand der vorliegenden Beschreibung ist. Nach dem Verfahren der WO 95/31553 wird das α-1,4-D-Glucan mittels eines biokatalytischen Verfahrens aus Saccharose unter Einwirkung eines Enzyms mit Amylosucrase-Aktivität, insbesondere mit einer Amylosucrase aus Bakterien der Spezies *Neisseria polysaccharea*, hergestellt. Diese Enzyme katalysieren die Bildung von α-1,4-glykosidisch verknüpften Glucanen, indem sie unter Freisetzung von D-Fructose den Glucosylrest des Saccharosemoleküls gemäß dem folgenden Reaktionsschema

$$\text{Saccharose} + (\alpha\text{-1,4-D-Glucosyl})_n \rightarrow \text{D-Fructose} + (\alpha\text{-1,4-D-Glucosyl})_{n+1}$$

auf die wachsende Polymerkette übertragen.

Ein besonders bevorzugtes Verfahren zur Gewinnung wasserunlöslicher linearer α-1,4-D-Glucane auf Grundlage des obigen Reaktionsschemas wird in der älteren, nicht vorveröffentlichten deutschen Patentanmeldung DE-A-198 27 978.1 beschrieben, deren Offenbarungsgehalt ausdrücklich Gegenstand der vorliegenden Beschreibung ist. Hierbei werden die wasserunlöslichen linearen α-1,4-D-Glucane aus Saccharose mittels Enzymen mit Amylosucrase-Aktivität, bevorzugt aus *Neisseria polysaccharea*, in wässrigen, pufferfreien Systemen synthetisiert. Die Reaktion kann auch in Gegenwart eines wasserlöslichen linearen oder verzweigten α-1,4-D-Glucans, beispielsweise eines wasserlöslichen Dextrins oder einer wasserlöslichen Amylose durchgeführt, da solche Glucane als Glucosylgruppenakzeptoren wirken, an denen das Enzym eine α-1,4-Glucankettenverlängerung katalysiert.

Bei einer solchen Kettenverlängerung entstehen auch aus verzweigten Polysacchariden wasserunlösliche lineare Polysaccharide im Sinne der vorliegenden Erfindung, da der Verzweigungsgrad des Glucosylgruppenakzeptors mit zunehmender Kettenverlängerung, also zunehmendem Polymerisationsgrad stark abnimmt. Zu diesem Zweck wird die Saccharose in großem molaren Überschuß zum Akzeptor eingesetzt. Auf diese Weise lassen sich α-1,4-D-Glukane mit einem Molekulargewicht im Bereich von $0{,}75 \times 10^2$ g/mol bis $10^7$ g/mol herstellen. Die linearen oligomeren oder polymeren Akzeptoren können dabei entweder von außen zugesetzt werden, sie können jedoch auch durch die Amylosucrase selbst aus Saccharose erzeugt werden.

Die Bildung der resistenten Stärke erfolgt durch Retrogradierung der nichtresistenten wasserunlöslichen linearen α-1,4-D-Glucane.

Die Retrogradierung der α-1,4-D-Glucane kann nach an sich bekannten Verfahren erfolgen, beispielsweise durch Erhitzen oder Extrusion.

Ein besonders bevorzugtes Verfahren zur Herstellung resistenter Stärke wird in der älteren, nicht vorveröffentlichten DE-A-198 30 618 beschrieben, auf die hier ausdrücklich Bezug genommen wird und deren Offenbarung Gegenstand der vorliegenden Beschreibung bildet. Dieses Verfahren ist einfach durchzuführen und liefert α-1,4-D-Glucanpräparationen mit einem hohen Anteil resistenter Stärke.

In einer vorteilhaften Ausführungsform entsprechend der genannten DE-A-198 30 618 erfolgt die Herstellung der resistenten Stärke dadurch, daß zunächst eine wäßrige Suspension oder Dispersion der α-1,4-D-Glucane hergestellt wird.

Diese Suspension oder Dispersion wird anschließend erwärmt, beispielsweise auf eine Temperatur von 50°C bis 100°C, und dann wird der erhaltene Kleister auf eine Temperatur von vorzugsweise im Bereich von 35°C bis zum Gefrierpunkt abgekühlt, bei der das α-1,4-D-Glucan retrogradiert, und dann gegebenenfalls getrocknet.

Die Begriffe "Suspension", "Dispersion" und Kleister haben hierbei die dem Fachmann geläufige Bedeutung (s.a. Römpp, Chemie-Lexikon, 9. Auflage, Thieme-Verlag).

In einer anderen Ausführungsform entsprechend der DE-A-198 30 618 erfolgt die Herstellung der resistenten Stärke dadurch, daß die wäßrige Suspension oder Dispersion der α-1,4-D-Glucane eingefroren wird, wobei das α-1,4-D-Glucan retrogradiert wird. Nach dem Auftauen wird dann gegebenenfalls getrocknet oder entwässert.

Die vollständige oder teilweise Retrogradierung der wasserunlöslichen linearen α-1,4-D-Glucane und die Bildung resistenter Stärke kann erfolgen, bevor die α-1,4-D-Glucane mit den Lebens- und Futtermitteladditiven zusammengegeben werden, sie kann während des Zusammenbringens der Bestandteile der Zusammensetzung erfolgen oder sie kann in der Mischung aus wasserunlöslichen linearen α-1,4-D-Glucanen, Additiven und gegebenenfalls weiteren Bestandteilen erfolgen.

Unter Lebens- und Futtermitteladditiven werden hier sowohl Stoffe als auch Mikroorganismen verstanden, die Lebens- oder Futtermitteln zur Beeinflussung oder zur Erzielung bestimmter Eigenschaften oder Wirkungen zugesetzt werden. Darunter fallen insbesondere Additive, die einen positive Effekt auf die Gesundheit und das Wohlbefinden von Mensch und Tier haben.

Die Lebens und Futtermitteladditive können Probiotika, Prebiotika oder sonstige Zusatzstoffe sein.

Unter Probiotika werden nicht-pathogene Mikroorganismen verstanden, die dem Lebens- oder Futtermittel lebend oder in Sporenform zugesetzt werden und die die Darmflora positiv beeinflussen können.

Beispiele für als Probiotika geeignete Mikroorganismen sind insbesondere Bakterien und Pilze. Bevorzugte Mikroorganismen sind Milchsäurebakterien, insbesondere der Gattung Lactobacillus, beispielsweise Bifidobakterien, Mikroorganismen der Gattung Streptococcus, beispielsweise Enterococcus-Stämme, und Hefen, beispielsweise der Gattung Saccharomyces, insbesondere der Spezies Saccharomyces boulardii.

Unter Prebiotika werden nicht verdauliche Stoffe verstanden, die dem Lebens- oder Futtermittel zugesetzt werden und die das Wachstum von bestimmten Bakterien im Dickdarm fördern.

Beispiele für Prebiotika sind insbesondere Ballaststoffe, beispielsweise Nahrungsfasern wie Oligo- und Polysccharide, beispielsweise Inulin, Oligofruktosen, Oligofructane und Fructo-Oligosaccharide.

Sonstige Zusatzstoffe sind Vitamine und Provitamine, insbesondere Vitamin A, die Vitamine $B_1$, $B_2$, $B_3$, $B_5$, $B_6$, $B_9$ und $B_{12}$, VitaminC, Vitamin D, Vitamin E, Vitamin F und Vitamin K; Antioxidantien; Öle, Fette und Fettsäuren, insbesondere mehrfach ungesättigte Fettsäuren, beispielsweise $\Omega$-3-Fettsäuren oder essentielle Fettsäuren wie Linol- und Linolensäure; sowie Kräuter und Extrakte.

Bevorzugt können Kombinationen von α-1,4-D-Glucanen und/oder resistenter Stärke und Lebens- und Futtermitteladditiven eingesetzt werden, die einerseits die technischen Voraussetzungen zur Verarbeitung des Lebensmittels nicht verändern und andererseits das Eigenschaftsbild des Lebensmittels nicht wesentlich beeinflussen. Dies ist zum Beispiel der Fall, wenn der kalorische Haushalt des Lebensmittels durch die funktionellen Additive nicht verändert wird. Dies wird entweder erreicht, indem beide oder mehrere funktionelle Additive gegenüber speziellen Eigenschaften neutral sind oder der positive oder negative Effekt des einen funktionellen Inhaltsstoffs durch den negativen oder positiven Effekt des anderen funktionellen Inhaltsstoffs gerade aufgehoben wird.

Besonders vorteilhaft sind Zusammensetzungen, die neben dem wasserunlöslichen linearen α-1,4-D-Glucan und/oder der resistenten Stärke, die selbst ein Prebiotikum darstellt, Probiotika enthalten. Bevorzugt ist das Probiotikum ein Bifidobakterium. Diese Kombination führt zu einem unerwartete symbiotischen Effekt, indem die resistente Stärke von den intestinalen Mikroorganismen unter Bildung kurzkettiger Fettsäuren verdaut wird, die wiederum als Nährstoff für die Bifidobakterien dienen und deren Proliferation fördern.

Die Herstellung der erfindungsgemäßen Zusammensetzungen kann durch einfaches Mischen erfolgen.

In einer besonders vorteilhaften Ausführungsform dient das wasserunlösliche lineare α-1,4-D-Glucan oder die daraus erhältliche resistente Stärke als Träger für wenigstens ein Lebens- oder Futtermitteladditiv.

In einer weiteren bevorzugten Ausführungsform liegt das wasserunlösliche lineare α-1,4-D-Glucan oder die daraus erhältliche resistente Stärke in Form von Mikropartikeln, insbesondere sphärischen Mikropartikeln vor, die ganz oder teilweise aus dem Glucan bestehen. Dies ist besonders vorteilhaft, wenn das wasserunlösliche lineare α-1,4-D-Glucan oder die daraus erhältliche resistente Stärke als Träger für das Lebens- und Futtermitteladditiv dienen soll.

Unter sphärischen Mikropartikeln werden hierbei annähernd kugelförmige Mikropartikel verstanden, deren Abweichung in den Achsenlängen vom Idealzustand einer Kugel, die durch von einem gemeinsamen Ursprung ausgehende, in den Raum gerichtete Achsen gleicher Länge, die den Radius der Kugel in allen Raumrichtungen definieren, beschrieben wird, nicht mehr als 40% beträgt. Bevorzugt werden sphärische Mikropartikel mit Abweichungen von nicht mehr als 25%, besonders bevorzugt nicht mehr als 15% verwendet.

Die spezifische Oberfläche der Mikropartikel liegt zweckmäßig in einem Bereich von 1 m²/g bis 100 m²/g, bevorzugt von 1,5 m²/g bis 20 m²/g und besonders bevorzugt von 3 m²/g bis 10 m²/g.

Der mittlere Durchmesser (Zahlenmittelwert) der Mikropartikel liegt zweckmäßig in einem Bereich von 1nm bis 100 μm, bevorzugt von 100 nm bis 10 μm und besonders bevorzugt von 1 μm bis 5 μm.

Die Dispersität D = $d_w/d_n$ der Mikropartikel, worin $d_w$ den Gewichtsmittelwert des Durchmessers und $d_n$ den Zahlenmittelwert des Durchmessers der Mikropartikel bedeutet, liegt zweckmäßig in einem Berich von 1 bis 10, vorzugsweise von 1,5 bis 5 und bevorzugt von 2 bis 3. Die Mittelwerte $d_w$ und $d_n$ sind definiert als

$$d_n = \Sigma \; n_i \; x \; d_i/\Sigma \; n_i;$$

und

$$d_w = \Sigma n_i \; x \; d_i^2/\Sigma \; n_i \; x \; d_i$$

worin

$d_i$     den Durchmesser der Partikel der Spezies i bedeutet;

$n_i$     die Anzahl der Partikel i mit dem Durchmesser $d_i$; und

i     einen fortlaufenden Parameter bedeutet.

Der Begriff Gewicht steht in diesem Zusammenhang nicht für Masse sondern für ein gewichtetes Mittel, wodurch die größeren Durchmesser einen höheren Stellenwert erhalten. Durch den Exponenten 2 werden Durchmesser größerer Partikel stärker gewichtet.

Die Bildung von Mikropartikeln, die ganz oder teilweise aus resisteter Stärke bestehen, kann beispielsweise durch einfaches Vermahlen der resistenten Stärke, die aus dem oben beschriebenen Kleister erhalten wurde, erfolgen.

Weitere Verfahren zur Herstellung von Mikropartikeln werden in den älteren deutschen Patentanmeldungen DE-A-197 37 481.6, DE-A-198 39 214.1, DE-A-198 39 216.9 und DE-A-198 39 212.5 beschrieben, auf die hier ausdrücklich Bezug genommen wird und deren Offenbarung ebenfalls Bestandteil der vorliegenden Beschreibung bildet.

Die Herstellung der bevorzugt sphärischen Mikropartikel erfolgt zweckmäßig durch Lösen der wasserunlöslichen α-1,4-D-Glucane oder der daraus erhältlichen resistenten Stärke in einem lebensmitteltechnologisch unbedenklichen Lösungsmittel, beispielsweise durch Lösen in wässrigem Alkali, Einbringen der Lösung in ein Fällmittel, vorzugsweise Wasser, Kühlen des dabei entstehenden Gemisches auf vorzugsweise 10°C bis -10°C und Abtrennen der gebildeten Mikropartikel.

Struktur und Oberfläche der Mikropartikel können durch die Art des Fällmittels gesteuert werden. Durch die Mitverwendung geeigneter, insbesondere in der Lebensmitteltechnologie zugelassener Zusatzstoffe, beispielsweise Zuckern wie Fructose, Saccharose und Glucose, kann ebenfalls Einfluß auf Struktur, Größe und Oberfläche der Partikel genommen werden.

Die Konzentration des α-1,4-D-Glucans oder der resistenten Stärke in der Lösung kann in einem weiten Bereich variieren und beträgt vorzugsweise ungefähr 0,1 bis 1 g pro ml Lösungsmittel.

Mikropartikel mit einer mittleren Größe von 0,1 μm bis 3 μm lassen sich bei diesem Verfahren vorteilhaft erhalten, wenn man dem Fällmittel ein heißwasserlösliches α-D-Glucan zusetzt.

Die Porosität der Mikropartikel läßt sich auch durch die Wahl des Verfahrens zur Herstellung des wasserunlöslichen linearen α-1,4-D-Glucans steuern. So kann beispielsweise der Zusatz von Hilfsstoffen bei der biotechnologischen Herstellung der Polysaccharide die Porosität der aus solchen Polysacchariden erhaltenen Mikropartikel beeinflussen. Insbesondere wurde gefunden, daß die Porosität von Mikropartikeln, die ganz oder teilweise aus den wasserunlöslichen linearen α-1,4-D-Glucanen gebildet werden, erhöht werden kann, wenn die Herstellung des Glucans aus Saccharose mittels Amylosucrase in Gegenwart eines Glucosylgruppenakzeptors, beispielsweise Dextrin, erfolgt. Dabei werden die Mikropartikel umso poröser, je höher die Konzentration des Glucosylgruppenakzeptors bei der Biotransformation ist.

Das Aufbringen der Lebens- und Futtermitteladditive auf das Trägermaterial kann beispielsweise bei flüssigen Lebens- und Futtermitteladditiven wie ungesättigten Fettsäuren durch einfaches Mischen erfolgen. Die Additive können aber auch dem Fällbad zugegeben werden, aus dem man die Mikropartikel gewinnt. Bei der Bildung der Mikropartikel adsorbieren die Additive dann auf der Oberfläche der Partikel.

In einer weiteren vorteilhaften Ausführungsform werden die Lebens- und Futtermitteladditive mit den wasserunlöslichen linearen α-1,4-D-Glucanen oder der resistenten Stärke wenigstens teilweise ummantelt oder verkapselt.

Auf diese Weise wird nicht nur die synergistische oder symbiotische Wirkung verstärkt, sondern auch ein zusätzlicher stabilisierender Effekt erreicht. Besonders vorteilhaft ist diese Ausführungsform bei einer Kombination von wasserunlöslichen linearen α-1,4-D-Glucanen und/oder resistenter Stärke mit Probiotika, wobei die lebenden Organismen mittels einer Schicht aus resistenter Stärke und gegebenenfalls anderen Polysacchariden verkapselt und dadurch auch vor schädlichen Einflüssen, die beispielsweise während der Verarbeitung von Nahrungsmitteln auftreten, geschützt werden.

Die Ummantelung kann in an sich bekannter Weise durch wäßrige Dispersions- oder Suspensionsprozesse erfolgen.

Die Zusammensetzungen können ausschließlich aus den wasserunlöslichen linearen α-1,4-D-Glucanen und/ oder den daraus erhältlichen resistenten Stärken und den jeweiligen Additiven bestehen oder weitere Zusatzstoffe, beispielsweise weitere Nahrungsergänzungsmittel enthalten.

Die erfindungsgemäßen Zusammensetzungen können die wasserunlöslichen linearen α-1,4-D-Glucane in nicht α-Amylase-resistenter Form, in Form der daraus erhältlichen resistenten Stärke oder in Form von Mischungen daraus enthalten.

In einer möglichen Ausführungsform enthält die erfindungsgemäße Zusammensetzung die Glucane überwiegend als wasserunlösliche lineare α-1,4-D-Glucane, also in nicht α-Amylase-resistenter Form. Solche Zusammensetzungen eignen sich beispielsweise zur Herstellung von Lebensmitteln und Lebensmittelvorprodukten, in denen die resistente Stärke erst durch eine Weiterbehandlung oder eine Verarbeitung vor dem Verzehr, beispielsweise durch Erwärmen, gebildet wird.

In einer weiteren Ausführungsform enthält die erfindungsgemäße Zusammensetzung die Glucane überwiegend in Form resistenter Stärke. Solche Zusammensetzungen können den Nahrungsmitteln beispielswise auch nach Verarbeitungsschritten wie Erwärmen oder Erhitzen zugegeben werden. In solchen Zusammensetzungen wird der Gehalt an resistenter Stärke, bezogen auf die Gesamtmenge an α-1,4-D-Glucan, möglichst hoch gewählt. Zweckmäßig beträgt der Gehalt, wie bestimmt nach Englyst (siehe supra), wenigstens 25 Gew.-%, bevorzugt wenigstens 65 Gew.-%, insbesondere 75%, und besonders bevorzugt wenigstens 90 Gew.-%, insbesondere 95 bis 99 Gew.-% und mehr.

Die erfindungsgemäßen Zusammensetzungen können in einer Vielzahl von Nahrungsmitteln eingesetzt werden. Beispiele für geeignete Lebensmittel sind Milch und Milchprodukte wie Yoghurt, Quark oder Schnitten und Pudding; Brot, Aufstriche, Getränke, Müsliriegel, Cerealien, Kekse, Kuchen, Gebäck, Soßen, Nudeln, Kartoffelpüree und andere Kartoffelgerichte wie Pommes Frites, Aufläufe, Verdicker, Designer Drinks, Getränkepulvern und Fertiggerichten.

Resistente Stärke auf Basis wasserunlöslicher linearer α-1,4-D-Glucane und ihre Zusammensetzungen können als Ersatzstoff für Nahrungsmittelinhaltsstoffe verwendet werden, insbesondere als Fettstoffersatz, und damit als Kalorienreduktionsmittel dienen. Sie können auf diese Weise eine Zellstimulation bewirken und den Appetit beeinflussen.

Der Abbau der resistenten Stärke im Darm zu kurzkettigen Fettsäuren, insbesondere zu besonders vorteilhaftem Butyrat, ihre Eignung als Ersatzstoff und die synergistischen und symbiotischen Effekte in Kombination mit anderen Lebens- oder Futtermitteladditiven sind jedoch nicht der einzige Vorteil bei der Verwendung resistenter Stärke in Nahrungsmitteln. Resistente Stärke kann nämlich insbesondere zusammen mit anderen Lebens- oder Futtermitteladditiven auch dazu benutzt werden, die Eigenschaften der Lebensmittel zu verbessern. So kann die erfindungsgemäße Zusammensetzung, abhängig von der Wahl der Additive, zu einer Erhöhung der Gelbildung, einer Verbesserung der Fließeigenschaften, einer Erhöhung oder Erniedrigung der Viskosität, einer Verbesserung des Sorptionsverhaltens, einer Erhöhung oder Verringerung der Quelleigenschaften und einer Verbesserung der Verkleisterungstemperatur von Produkten führen, die Polysaccharide enthalten. Ebenso kann die erfindungsgemäße Zuammensetzung eingesetzt werden, um die Löslichkeit, die Transparenz und die Textur der Kleisterstruktur zu beeinflussen und die Wärme-, Kälte-, Säure- und Scherstabilitäten der Produkte zu verbessern. Ferner kann der Einsatz der erfindungsgemäßen Zusammensetzungen die Filmbildungstendenz erhöhen, die Gefrier- und Taustabilitäten positiv beeinflussen und die Verdaulichkeit verbessern. Daneben können auch kaschierende Effekte zum Tragen kommen, die sich auf den geschmack, das sog. Mundgefühl (mouth feel) oder den Geruch begründen.

Die Tatsache, daß die aus wasserunlöslichen linearen α-1,4-D-Glucanen erhältlichen resistenten Stärken bei der Verdauung einen überaus hohen Gehalt an für die Darmflora günstigen Butyraten liefert, macht diese Stärke auch für die Verwendung in medizinischen und veterinärmedizinischen Präparaten geeignet, insbesondere in Kombination mit anderen funktionellen Additiven, beispielsweise den oben erwähnten Lebens- und Futtermitteladditiven, aber auch zusammen mit anderen Heilmitteln. Die Verwendung von resistenter Stärke auf Basis wasserunlöslicher linearer α-1,4-D-Glucane als Arzneimittel, allein oder in Kombination mit anderen funktionellen Additiven, wirkt sich auf zahlreiche Krankheitsbilder positiv aus. So eignen sich Arzneimittel auf Basis der erfindungsgemäßen resistenten Stärke beispielsweise zur Behandlung von Herzerkrankungen, Magen-Darm-Erkrankungen, Arthritis, immunologischen Erkrankungen, mentalen Dysfunktionen, Nervenerkrankungen, psychischen Störungen, Schlafstörungen, Erkrankungen der Muskulatur, Krankheiten, die den Hormonhaushalt beeinflussen, Schilddrüsenerkrankungen, Krankheiten der inneren Organe, Veränderungen des Blutbilds, Kreislauferkrankungen, Allergien, Hauterkrankungen und Erkrankungen, die auf Mangelernährung zurückzuführen sind. Ebenso können Präparate mit resistenter Stärke als Appetitzügler eingesetzt werden.

Dementsprechend ist resistente Stärke auf Basis wasserunlöslicher α-1,4-D-Glucane, allein oder in Kombination mit anderen funktionellen Additiven, auch als Arzneimittel einsetzbar, und zwar sowohl als Therapeutika als auch als Prophylaktika oder Diagnostika. Die medizinischen, resistente Stärke enthaltenden Präparate können hierbei übliche Hilfs- und Trägerstoffe enthalten und in den üblichen Darreichungsformen, beispielsweie in Form von Tabletten, Depot-Formulierungen oder Mitteln mit kontrollierter Wirkstofffreisetzung vorliegen.

Die vorliegende Erfindung wird durch die nachfolgenden Beispiele näher erläutert:

Beispiele

Beispiel 1

Herstellung von α-1,4-D-Glucanen

In ein 5-I-Gefäß wurden 5 I einer sterilisierten 30%igen Saccharose Lösung gegeben. Ein Enzymextrakt, der eine Amylosucrase aus Neisseria polysaccharea enthielt (WO-A-95/31553), wurde in einer Portion zugegeben und es wurde gemischt. Die eingesetzte Enzymaktivität betrugt in diesem Experiment 148000 Units. Das verschlossene Gefäß wurde bei 37°C inkubiert. Während der Dauer der Biotransformation bildete sich ein weißer Niederschlag. Die Reaktion wurde nach 39 h beendet. Der Niederschlag wurde abzentrifugiert, bei -70°C eingefroren und anschließend gefriergetrocknet. Die Masse des gefriergetrockneten Feststoffes betrug 526,7 g (70,2 % Ausbeute).

Zur Abtrennung niedermolekularer Zucker wurden 200 g des Feststoffes mit Wasser 30 min unter Rühren bei Raumtemperatur gewaschen, bei -70 °C eingefroren und gefriergetrocknet. Der Gehalt an Fruktose und Saccharose wurde nach Lösen des Feststoffes in DMSO durch einen gekoppelten enzymatischen Assay bestimmt und betrug 4,61 mg Fruktose pro 100 mg Feststoff (4, 6 %). Der Gehalt an Saccharose lag unter der Nachweisgrenze.

Der Überstand der Biotransformation wurde bei 95°C denaturiert. Nach Abkühlen auf Raumtemperatur wurde erneut zentrifugiert. Der klare Überstand wurde bei -70°C eingefroren und über 3 Tage bei 4 °C aufgetaut. Der so erzeugte Niederschlag wurde bei -70 °C eingefroren und gefriergetrocknet.

Zur Abtrennung niedermolekularer Zucker wurden 39,5 g des Feststoffes mit Wasser 30 min unter Rühren bei Raumtemperatur gewaschen, bei -70°C eingefroren und gefriergetrocknet. Der Gehalt an Fruktose und Saccharose wurde nach Lösen des Feststoffes in DMSO durch einen gekoppelten enzymatischen Assay gemäß STITT et al. (Meth. Enzym., 174 (1989) 518 - 552) bestimmt und betrug 2,27 mg Fruktose pro 100 mg Feststoff. Der Gehalt an Saccharose lag unter der Nachweisgrenze.

Beispiel 2

Bestimmung des Molekulargewichts des nach Beispiel 1 erhaltenen Materials

Es wurden 2 mg des α-1,4-D-Glucans aus Beispiel 1 bei Raumtemperatur in Dimethylsulfoxid (DMSO, p.a. von Riedel-de-Haen) gelöst und filtriert. Ein Teil der Lösung wurde in eine Säule zur Gelpermeationschromatographie injiziert. Als Elutionsmittel wurde DMSO verwendet. Die Signalintensität wurde mittels eines RI-Detektors gemessen und gegen Pullulanstandards (Firma Polymer Standard Systems) ausgewertet. Die Flußrate beträgt 1.0 ml pro Minute.

Die Messung ergibt ein Zahlenmittel des Molekulargewichts (Mn) von 2.326 g/mol und ein Gewichtsmittel des Molekulargewichts (Mw) von 3.367 g/mol. Die Wiederfindungsrate beträgt 100 %.

Beispiel 3

Bestimmung des RS-Gehaltes

200 mg (Trockengewicht) des auf seinen RS-Gehalt zu analysierenden pulverförmigen Produktes wurden nach der Methode von Englyst et al. (Eur. J. Clin. Nutrition, 46 (1992) (Suppl. 2) S 33-550) zur Bestimmung des RS-Gehaltes mit der beschriebenen Enzymmischung bei pH 5,2 120 min inkubiert. Nach Beendigung des enzymatischen Abbaus wurde die Aktivität der Enzyme durch Erniedrigung des pH-Wertes auf einen Wert von 3 und der Temperatur auf 20°C gestoppt. Anschließend erfolgte durch Zugabe der 4-fachen Menge an Ethanol die Einstellung einer 80%igen (v/v) ethanolischen Lösung. Die 80%ige ethanolische Lösung wurde 1 h bei Raumtemperatur stehengelassen. Das Präzipitat wurde zentrifugiert (2500 x g, 10 min) und der Überstand verworfen. Der Rückstand wurde dreimal mit 80%igem (v/v) Ethanol und einmal mit absolutem Ethanol gewaschen und zentrifugiert. Der Rückstand wurde lyophilisiert und gewogen. Die Trockenmasse des Rückstandes wurde bestimmt und der RS-Gehalt nach folgender Gleichung berechnet:

$$RS\ [\%] = 100\ x\ \text{Gewicht des Rückstandes (Trockengewicht)/Einwaage}$$

(Trockengewicht)

Beispiele 4 bis 7

Ein lineares, naturidentisches $\alpha$-1,4-D-Glukan nach Beispiel 1 wurde in wässriger Lösung erhitzt und ein Kleister gebildet. Dieser Kleister wurde auf 10 Gew.-% Feststoffanteil eingestellt und portioniert. Die Portionen wurden bei 4 und 25°C (Beispiel 5 bzw. 6) oder mit Hilfe eines Stufenprogramms (Beispiel 7) retrogradiert. Des weiteren wurde das lineare Kohlenhydratpolymer aus dem Reaktionsansatz ausgefroren (Beispiel 4). Die retrogradierten Muster wurden getrocknet und die Bestimmung des RS-Gehaltes wie oben beschrieben durchgeführt.
Tabelle 1 illustriert den Einfluß der Retrogradationstemperatur und -bedingungen auf den RS-Gehalt im Produkt, hergestellt aus einem 10-proz. Kleister der verwendeten $\alpha$-1,4-D-Glucane durch 24-stündige Retrogradation.

Tabelle 1

| Beispiel | Retrogradationstemperatur | RS (Gew.-%) |
|----------|---------------------------|-------------|
| 4 | -70°C | 78 ± 4 |
| 5 | 4°C | 70 ± 2 |
| 6 | 25°C | 87 ± 1 |
| 7 | Stufenprogramm | 74 ± 3 |

Das Beispiel in Tabelle 1 zeigt, daß die Retrogradationstemperatur den RS-Gehalt beeinflußt. So führt eine Retrogradation bei 25 °C zu einem deutlich höheren RS-Anteil verglichen mit einer Retrogradation bei 4 °C. Durch Retrogradation bei -70 °C erhält man hingegen einen leicht höheren RS-Anteil verglichen mit dem nach Retrogradation bei 4°C.

Beispiel 8

Bestimmung der Löslichkeit der von $\alpha$-1,4-D-Glucanen und Klassifizierung nach Deutschem Arzneimittelbuch (DAB)

564 mg $\alpha$-1,4-D-Glucan aus Beispiel 1 wurden in ca. 0,5 l bidestilliertem Wasser bei 1,3 bar und 130 °C für 1,5 Stunden in einem Autoklaven erhitzt (Apparat Certoclav). Von dem Reaktionsgefäß ist zuvor das Gewicht gemessen worden. Danach wird die Apparatur entspannt und bei Raumtemperatur abgekühlt. Der Inhalt wird gewogen. Er entspricht 501,74 g. Nach weiteren 24 Stunden wird zentrifugiert und dekantiert. Der feste Rückstand wird getrocknet und ausgewogen. Es sind 468 mg. Daraus errechnet sich ein gelöster Anteil von 96 mg. Bezogen auf das eingesetzte Lösungsmittel errechnet sich daraus, daß für 1 mg $\alpha$-1,4-D-Glucan 5226 mg Wasser notwendig sind. Gemäß der Klassifizierung nach Deutschem Arzneimittelbuch ergibt sich daraus die Einteilung, daß diese Substanz "sehr schwer löslich" ist, da zwischen 1.000 und 10.000 Teilen Lösungsmittel notwendig sind, um 1 Teil der Substanz in Lösung zu bringen. Dies entspricht der Löslichkeitsklasse 6 nach DAB.

Beispiel 9

Bestimmung der Löslichkeit von $\alpha$-1,4-D-Glucanen und Klassifizierung nach Deutschem Arzneimittelbuch (DAB)

Der Versuch wurde mit einem auf ähnliche Weise wie in Beispiel 1 erhaltenen $\alpha$-1,4-D-Glucan entsprechend Beispiel 8 durchgeführt. Den einzigen Unterschied bildete ein Kühlprozeß, der nach der Autoklavbehandlung und dem Abkühlen auf Raumtemperatur nachgeschaltet wurde. Das Substanzgemisch wird für 3 Stunden bei 5 °C aufbewahrt.
Es wurden 526 mg $\alpha$-1,4-D-Glucan auf ca. 480 ml bidestilliertem Wasser eingewogen. Nach der thermischen Behandlung ergab sich eine Auswaage von 468,09 g. Das getrocknete Sediment betrug 488 mg. Demnach waren 38 mg des $\alpha$-1,4-D-Glukan in Lösung gegangen. Dies entsprach einem Verhältnis von 1 mg Substanz zu 12.305 Teilen Lösungsmittel. Demnach war die Substanz nach dieser Behandlungsmethode in Klasse Nummer 7 nach DAB einzustufen und danach als praktisch unlöslich zu klassifizieren, weil mehr als 10.000 Teile Lösungsmittel für ein Teil Substanz benötigt wurden.

Beispiel 10

Herstellung von Müsliriegeln mit resistenter Stärke

Es wurden Müsliriegel mit wechselnden Mengen resistenter Stärke (RS) hergestellt.

| Grundrezept: | 40% | Honig |
|---|---|---|
| | 30% | Haferflocken |
| | 6% | Sonnenblumenkerne |
| | 9% | Haselnüsse |
| | 6% | Haferfleks |
| | 9% | Schokoladeblättchen |
| | | |
| a) | 40% | Honig |
| | 30% | Haferflocken |
| | 6% | Sonnenblumenkerne |
| | 9% | Haselnüsse |
| | 6% | Haferfleks |
| | 9% | Schokoladeblättchen |
| | 3% | Amylose (RS) |
| | | |
| b) | 40% | Honig |
| | 24% | Haferflocken |
| | 6% | Sonnenblumenkerne |
| | 9% | Haselnüsse |
| | 6% | Haferfleks |
| | 6% | Schokoladeblättchen |
| | 9% | Amylose (RS) |
| | | |
| c) | 36% | Honig |
| | 24% | Haferflocken |
| | 6% | Sonnenblumenkerne |
| | 9% | Haselnüsse |
| | 6% | Haferfleks |
| | 6% | Schokoladeblättchen |
| | 9% | Amylose (RS) |
| | 4% | Hochungesättigte Fettsäuren |
| | | |
| d) | 40% | Honig |
| | 21% | Haferflocken |
| | 6% | Sonnenblumenkeme |
| | 9% | Haselnüsse |
| | 6% | Haferfleks |
| | 6% | Schokoladeblättchen |
| | 12% | Amylose (RS) |

Die Zutaten wurden gut gemischt und ca. 4 Std. bei 70°C im Trockenschrank oder Ofen gebacken. Die Müsliriegel waren auch bei einem Gehalt an resistenter Stärke in ihrer Konsistenz von den Riegeln nach dem Grundrezept nicht zu unterscheiden und wohlschmeckend.

Beispiel 11

Untersuchung der Bildung kurzkettiger Fettsäuren (SCFA) durch in vitro-Fermentation der α-Amylase resistenten Anteile durch frisch entnommene Faecesproben.

1 ml einer 5%igen Faecessuspension (15g frisch entnommenen Humanfaeces in 50 ml Soerensen-Puffer, pH 6,5; Puffer aus Kaliumhydrogenphosphat und Dinatriumhydrogenphospat-Dihydrat) wurden in mit Stickstoff begasten Cryo-Röhrchen mit je 10 mg, durch enzymatische Hyrolyse isolierten, resistenten Strukturen entsprechend Beispiel 1 und, als Vergleich, von Novelose, retrogradierter Maisstärke, National Starch & Chemical, USA, gemischt und homogenisiert. Die Fermentation erfolgte bei 37°C. Es wurden stündlich Proben entnommen und eingefroren.

Die Konzentration an kurzkettigen Fettsäuren wurde in einer Faecessuspension mit Hilfe der Gaschromatographie auf einer Kapillarsäule (Carbowax 20M) unter Nutzung eines Temperaturprogramms bestimmt. Als GC-System wurde eine HP 5890 Series II-Station mit HP 7673 GC/SCF-Injektor, HP GC-Autosamplercontroller, Detektor FID; Software - HP Chemstation, verwendet. Helium wurde als mobile Phase benutzt.

200 mg der Faecessuspension wurden mit der vierfachen Menge Wasser suspendiert und homogenisiert. Von dieser verdünnten Arbeitsfaecessuspension wurde ein Teil für die Trockenmassebestimmung verwendet.

500 mg der Arbeitsfaecessuspension wurden zentrifugiert. 100 µl des Überstandes wurden mit 25 µl 1M Natriumhydroxidlösung versetzt. Das verschlossenen Gefäß wurde mit durchbohrtem Deckel in flüssigen Stickstoff gegeben und gefriergtrocknet. Die getrocknete Probe wurde mit 100 µl 5M Ameisensäure und 400 µl Aceton versetzt und auf einem Vortex geschüttelt. Die sich bildende organische Phase wurde in Vials eines Autosamplers dekantiert, die sofort verschlossen wurden. Aus ihnen wurde je 1 µl in eine GC injiziert. Als externe Standards wurden Essigsäure, Propionsäure, Buttersäure, iso-Butterssäure, Valeriansäure und iso-Valeriansäure (Sulpeco) verwendet.

Die Fermentation der resistenten Strukturen wurden parallel in Doppelfermentationsstudien durchgeführt.

Die Fermentierbarkeit der eingesetzten Strukturen erwies sich als unterschiedlich. Insbesondere unterschieden sich die Bildungsraten und die Spektren der kurzkettigen Fettsäuren.

Durch in vitro-Fermentation der resistenten Strukturen entsprechend Beispiel 1 wurden deutlich höhere Spiegel an kurzkettigen Fettsäuren und an Butyrat in vergleichbaren Fermentationszeiten erreicht. Während durch 8stündige Fermentation der resistenten Strukturen entsprechend Beispiel 1 ein SCFA-Spiegel von ca. 2000 µmol/g Trockengewicht eingestellt wurde, wobei der Butyratgehalt ca. 60% betrug, lag der SCFA-Spiegel nach gleicher Fermentationszeit resistenter Strukturen aus Novelose nur bei ca 750 µmol Trockengewicht. Das Butyrat war dabei mit einem Anteil von ca 35% vertreten. Durch die Fermentation resistenter Strukturen des erfindungsgemäß als Ausgangsprodukt eingesetzten wasserlöslichen α-1,4-D-Glucans wird also schneller und mehr Butyrat erzeugt als durch resistente Stärke von Novelose.

Beispiel 12

Einfluß einer Kombination von resistenter Stärke und Bifido Bakterien auf die Aktivität und das Wachstum von Bifidobakterien sowie Darmzellen

Zur Austestung des Einflusses der Kombination von resistenter Stärke und Bifido Bakterien auf die Darmflora wurden 10 g resistente Stärke, hergestellt wie in den Beispielen 4 bis 7 beschrieben, an 5 gesunde Probanden zur oralen Aufnahme über einen Zeitraum von 14 Tagen verabreicht. Die Proben wurden während des Frühstücks in Form eines Bifidobakterien enthaltenden Joghurts verzehrt, in den das Polyglucan eingerührt wurde (Joghurt $LC_1$ der Firma Nestlé). Die Probanden hatten ein Durchschnittsalter von 38,9 und ein durchschnittliches Gewicht von 67,3 kg. Die Untersuchungswerte wurden mit einer Kontrolle verglichen. Die Kontrollgruppe bestand aus den gleichen Probanden. Sie wurden 3 Monate vor der zweiten Untersuchung untersucht. Es wurde Joghurt mit Bifido Bakterien ohne RS aufgenommen. Bereits 10 Wochen vor dem Beginn der Kontrolluntersuchungung und in den etwa 10 Wochen von der Beendigung der ersten Kontrolluntersuchung bis zur zweiten Untersuchung (RS-Bifido) haben die Probanden keinen Joghurt oder ähnliche Lebensmittel zu sich genommen, die Bifido Bakterien enthalten.

Die absolute Menge an Bifido Bakterien wurde gemäß einer erstmals in der folgenden Literatur publizierten Methode bestimmt ("A Color Atlas of Anaerobic Bacteria", 1984, Seiten 53 - 65, T. Mitsuoka (Hrsg.), Verlag Kabushiki Kaisha Sobunsha, Tokyo, Japan (1984)). Die gewachsenen Kolonien wurden in unterschiedlichen Medien kultiviert, nach dem Genotyp beurteilt und ausgezählt. Die Gesamtsumme aller Mikroorganismen wurde als die Gesamtsumme der Mikroorganismen jedes einzelnen Probanden angenommen. Die relative Anzahl der Bifido Bakterien wurde bestimmt, in dem die Zahl bei der Auszählung der Bifido Bakterien durch die Gesamtsumme dividiert und mit 100 multipliziert wurde.

Die relative Veränderung der Gesamtsumme der Bifido Bakterien wurde dadurch berechnet, daß die Zahl der Bifidos pro Gramm Faeces mit dem Gewicht der Faeces multipliziert wurde. Diese Zahl zu Beginn der Untersuchung wurde gleich 100 gesetzt. Der Vergleichswert nach 14 Tagen wurde zu diesem Normwert jedes einzelnen Probanden in Beziehung gesetzt. Die Werte der Untersuchung sind in Tabelle 2 zusammengefaßt. Die relativen Anteile der Bifido Bakterien werden als Mittelwerte über die Zeit und die Probanden angegeben. Die relativen Anteile Bifido Bakterien in der Gesamtzahl der Mikroorganismen wird als Wert zur Basis 100, dem Wert vor der Behandlung mit $LC_1$ oder RS-Bifido, auf der Basis des 14-tägigen Endwerts angegeben. Die Ergebnisse zeigen, daß ein Anstieg der täglichen Faecesmenge auftritt und zwar ein höherer Anstieg bei Verwendung von RS-Bifido. Die pH-Werte werden ca. um 0,5 abgesenkt. Weiterhin ist der positive Einfluß auf die Darmepitheizellen an dem starken Anstieg kurzkettiger Fettsäuren erkennbar. Dieser Effekt wird mit den Bifidobakterien allein nicht beobachtet.

Tabelle 2

| | vor $LC_1$ | nach $LC_1$ | vor RS-Bifido | nach RS-Bifido |
|---|---|---|---|---|
| Faeces Gewicht (g/Tag) | 125+/-29 | 137+/-35 | 127+/-32 | 148+/-35 |
| relative Änderung der Faeces Masse | 100 | 110 | 100 | 116 |
| Faeces pH-Wert | 6,6+/-0,5 | 5,9+/-0,5 | 6,5+/-0,5 | 6,0+/-0,5 |
| Gesamtzahl der Mikroorganismen pro g Faeces | $8,5 \times 10^8$+/-0,2$^8$ | $9,2 \times 10^8$+/-0,2$^8$ | $8,6 \times 10^8$+/-0,2$^8$ | $11,2 \times 10^8$+/-0,2$^8$ |
| relativer Anteil der Bifido Bakterien (%) | 9,9+/-0,2 | 10,3+/-0,2 | 9,9+/-0,2 | 13,9+/-0,2 |
| relativer Anteil der Bifidobakterien in der Gesamtzahl der Mikroorganismen | 100 | 195 | 100 | 255 |
| Acetat µmol/g Trockengewicht Faeces* | 365+/-15 | 335+/-15 | 387+/-15 | 1021+/-15 |
| Propionat µmol/g Trockengewicht Faeces* | 87+/-15 | 91+/-15 | 89+/-15 | 675+/-15 |
| Butyrat µmol/g Trockengewicht Faeces* | 99+/-15 | 100+/-15 | 102+/-15 | 617+/-15 |

* nach 6 Stunden Fermentation (= Sättigungswert)

Beispiel 13

Herstellung von hier z.B. Polyglucan Plätzchen mit verringertem Kaloriengehalt.

a) Vergleichsbeispiel
20 g Zucker und 50 g weiche Butter werden schaumig geschlagen. Dann werden ein halbes Ei, 50 g Weizenmehl, 30 g gemahlene Haselnüsse, 1 Teelöffel, ein wenig Zitronenschalen, 1 Teelöffel Backpulver und 1 Teelöffel Vanillezucker hinzugegeben.
Die Masse wird gut gerührt, bis die Masse sehr trocken und krümelig ist. Es wird ein wenig Milch hinzugegeben und verrührt, so daß sich der Teig leicht aufnehmen läßt. Es wird je ein Teelöffel der Masse auf ein Backblech geben. Im vorgeheizten Backofen (Heißluft) werden die Kekse bei 175°C ca. 15 Minuten gebacken.
Herstellung von Backwaren mit Weizenmehl (Vergleichsbeispiel) und Polyglucan Plätzchen

b)
Die Durchführung erfolgt wie in Beispiel 13a anstelle des Zuckers (Saccharose) wurde ca. 20 g Polyglucan verwendet (inklusive 1 Teelöffel Vanillezucker). Um eine akzeptable Süße zu erreichen, wurde mit einem handelsüblichen Süßungsmittel (z.B. Natreen) in äquivalenter Dosierung gesüßt.
Testpersonen (8) bescheinigten, daß in dem mit dem Geschmack verbundenen Kriterien, wie Mundgefühl, crisp-Effekt, Konsistenz, Klebrigkeit, Beiß- und Kaueffekte und -gefühl und Süße, kein erkennbarer Unterschied empfunden werden konnte, bzw. wenn eine Differenz ausgemacht wurde, daß diese nicht als nachteilig gesehen worden ist. Die Backwaren in Form von Keksen sind wohlschmeckend.
Damit ist eine Einsatzmöglichkeit als 'bulking agent', als Zuckerersatzstoff, vor allem in Speisen, aber auch Getränken, etwa Milch-Getränke, Trinkyoghurts, gegeben.

**Patentansprüche**

1. Zusammensetzung, umfassend ein wasserunlösliches lineares $\alpha$-1,4-D-Glucan und/oder eine daraus erhältliche resistente Stärke, wobei linear bedeutet, daß der Verzweigungsgrad in 2-, 3- oder 6-Position den Wert 0,5% nicht überschreitet, und wenigstens ein weiteres Lebens- oder Futtermitteladditiv, ausgewählt aus der Gruppe bestehend aus Probiotika, Prebiotika, Vitaminen und Provitaminen, Antioxidantien, Ölen und Fetten und Fettsäuren, sowie Mischungen daraus.

2. Zusammensetzung nach Anspruch 1, worin das wenigstens eine weitere Lebens- oder Futtermitteladditiv ein Probiotikum, insbesondere ein Bifidobakterium ist.

3. Zusammensetzung nach einem der Ansprüche 1 oder 2, worin das wasserunlösliche lineare $\alpha$-1,4-D-Glucan und/oder die daraus erhältliche resistente Stärke als Trägermaterial für das wenigstens eine Lebens- und Futtermitteladditiv fungiert.

4. Zusammensetzung nach einem der Ansprüche 1 bis 3, worin das wasserunlösliche lineare $\alpha$-1,4-D-Glucan und/oder die daraus erhältliche Stärke in Form von Mikropartikeln vorliegt.

5. Zusammensetzung nach einem der Ansprüche 1 bis 4, worin das Lebens- oder Futtermitteladditiv von dem wasserunlöslichen linearen $\alpha$-1,4-D-Glucan und/oder der daraus erhältlichen resistenten Stärke wenigstens teilweise ummantelt ist.

6. Zusammensetzung nach Anspruch 5, worin das wasserunlösliche lineare $\alpha$-1,4-D-Glucan ein Molekulargewicht $M_w$ von 0,75 x $10^2$ bis $10^7$ g/mol, bevorzugt von $10^3$ bis $10^6$ g/mol und besonders bevorzugt von $10^3$ bis $10^5$ g/mol aufweisen.

7. Zusammensetzung nach einem der Ansprüche 1 bis 6 worin das wasserunlösliche lineare $\alpha$-1,4-D-Glucan erhältlich ist durch in-vitro Polymerisation von Glucose unter Einwirkung eines Enzyms mit Amylosucrase-Aktivität.

8. Zusammensetzung nach einem der Ansprüche 1 bis 7, welche ein Nahrungsergänzungsmittel ist.

9. Verwendung einer Zusammensetzung nach einem der Ansprüche 1 bis 8 zur Herstellung von Nahrungsmitteln oder Nahrungsmittelvorprodukten.

10. Nahrungsmittel oder Nahrungsmittelvorprodukt, erhältlich unter Verwendung einer Zusammensetzung nach einem der Ansprüche 1 bis 8.

11. Resistente Stärke auf Basis wasserunlöslicher linearer $\alpha$-1,4-D-Glucane als Arzneimittel, wobei linear bedeutet, daß der Verzweigungsgrad in 2-, 3- oder 6-Position den Wert 0,5% nicht überschreitet.

12. Resistente Stärke nach Anspruch 11, worin das Arzneimittel ein Magen-Darm-Mittel ist.

13. Pharmazeutische oder veterinärmedizinische Zusammensetzung, umfassend einen Gehalt an resistenter Stärke auf Basis wasserunlöslicher linearer $\alpha$-1,4-D-Glucane, wobei linear bedeutet, daß der Verzweigungsgrad in 2-, 3- oder 6-Position den Wert 0,5% nicht überschreitet.

14. Zusammensetzung nach Anspruch 13, ferner umfassend ein weiteres funktionelles Additiv.

15. Zusammensetzung nach Anspruch 14, worin das funktionelle Additiv ein Lebens- oder Futtermitteladditiv ist, insbesondere ein Probiotikum, beispielsweise ein Bifidobakterium.

16. Zusammensetzung nach Anspruche 15, worin das funktionelle Additiv ein medizinischer Wirkstoff, insbesondere ein Heilmittel ist.

17. Verwendung von resistenter Stärke auf Basis wasserunlöslicher linearer $\alpha$-1,4-D-Glucane zur Herstellung eines Arzneimittels zur Behandlung und/oder Vorbeugung von Magen-Darm-Erkrankungen, wobei linear bedeutet, daß der Verzweigungsgrad in 2-, 3- oder 6-Position den Wert 0,5% nicht überschreitet.

**Claims**

1. A composition comprising a water-insoluble linear $\alpha$-1,4-D-glucan and/or a resistant starch obtainable therefrom, linear meaning that the degree of branching in the 2, 3 or 6 position does not exceed the value 0.5 %, and at least one further food additive or feed additive selected from the group consisting of probiotics, prebiotics, vitamins and provitamins, antioxidants, oils and fats and fatty acids, and mixtures thereof.

2. The composition as claimed in claim 1, wherein the at least one further food additive or feed additive is a probiotic, in particular a bifido bacterium.

3. The composition as claimed in either claim 1 or 2, wherein the water-insoluble linear $\alpha$-1,4-D-glucan and/or the resistant starch obtainable therefrom acts as carrier material for the at least one food additive and feed additive.

4. The composition as claimed in one of claims 1 to 3, wherein the water-insoluble linear $\alpha$-1,4-D-glucan and/or the starch obtainable therefrom is present in the form of microparticles.

5. The composition as claimed in one of claims 1 to 4, wherein the food additive or feed additive is at least in part enveloped by the water-insoluble linear $\alpha$-1,4-D-glucan and/or the resistant starch obtainable therefrom.

6. The composition as claimed in claim 5, wherein the water-insoluble linear $\alpha$-1,4-D-glucan has a molecular weight $M_w$ of from 0.75 x $10^2$ to $10^7$ g/mol, preferably from $10^3$ to $10^6$ g/mol, and particularly preferably from $10^3$ to $10^5$ g/mol.

7. The composition as claimed in one of claims 1 to 6, wherein the water-insoluble linear $\alpha$-1,4-D-glucan is obtainable by in vitro polymerization of glucose in the presence of an enzyme having amylosucrase activity.

8. The composition as claimed in one of claims 1 to 7 which is a foodstuff supplement.

9. Use of a composition as claimed in one of claims 1 to 8 for producing foodstuffs or foodstuff precursors.

10. A foodstuff or foodstuff precursor obtainable with the use of a composition as claimed in one of claims 1 to 8.

11. A resistant starch based on water-insoluble linear $\alpha$-1,4-D-glucans as medicament, where linear means that the degree of branching in the 2, 3 or 6 position does not exceed the value 0.5 %.

12. The resistant starch as claimed in claim 11, wherein the medicament is a gastrointestinal composition.

13. A pharmaceutical or veterinary composition comprising a content of resistant starch based on water-insoluble linear $\alpha$-1,4-D-glucans, where linear means that the degree of branching in the 2, 3 or 6 position does not exceed the value 0.5 %.

14. The composition as claimed in claim 13, additionally comprising a further functional additive.

15. The composition as claimed in claim 14, wherein the functional additive is a food additive or feed additive, in particular a probiotic, for example a bifido bacterium.

16. The composition as claimed in claim 15, wherein the functional additive is a medicinal compound, in particular a therapeutic agent.

17. The use of resistant starch based on water-insoluble linear $\alpha$-1,4-D-glucans for producing a medicament for treating and/or preventing gastrointestinal disorders, where linear means that the degree of branching in the 2, 3 or 6 position does not exceed the value 0.5 %.

**Revendications**

1. Composition comprenant un $\alpha$-1,4-D-glucane linéaire insoluble dans l'eau et/ou un amidon résistant obtenu à partir de celui-ci, dans lequel linéaire signifie que le degré de ramification en position 2-, 3- ou 6- ne dépassé pas la valeur 0,5 %, et au moins un autre additif de denrées alimentaires ou de matières fourragères, choisi dans le groupe des probiotiques, prébiotiques, vitamines et provitamines, des anti-oxydants, huiles et graisses et acides gras, ainsi que leurs mélanges.

2. Composition selon la revendication 1, dans laquelle le au moins un autre additif de denrées alimentaires ou de matières fourragères est un probiotique, en particulier un bifidobacterium.

3. Composition selon une des revendications 1 ou 2, dans laquelle l'$\alpha$-1,4-D-glucane linéaire insoluble dans l'eau et/ou l'amidon résistant obtenu à partir de celui-ci fonctionne comme matériau support pour le au moins un additif de denrées alimentaires ou de matières fourragères.

4. Composition selon une des revendications 1 à 3, dans laquelle l'$\alpha$-1,4-D-glucane linéaire insoluble dans l'eau et/ou l'amidon résistant obtenu à partir de celui-ci se présente sous la forme de microparticules.

5. Composition selon une des revendications 1 à 4, dans laquelle l'additif de denrées alimentaires ou de matières fourragères est enrobé au moins partiellement par l'$\alpha$-1,4-D-glucane linéaire insoluble dans l'eau et/ou l'amidon résistant obtenu à partir de celui-ci.

6. Composition selon la revendication 5, dans laquelle l'$\alpha$-1,4-D-glucane linéaire insoluble dans l'eau présente une masse moléculaire $M_w$ de 0,75 x $10^2$ à $10^7$ g/mol, de préférence de $10^3$ à $10^6$ g/mol et mieux encore de $10^3$ à $10^5$ g/mol.

7. Composition selon une des revendications 1 à 6 dans laquelle on peut obtenir l'$\alpha$-1,4-D-glucane linéaire insoluble dans l'eau par polymérisation *in vitro* de glucose sous l'action d'une enzyme avec une activité amylosucrase.

8. Composition selon une des revendications 1 à 7, qui est un agent de complément alimentaire.

9. Utilisation d'une composition selon une des revendications 1 à 8 pour la préparation de précurseurs de denrées alimentaires ou de matières fourragères.

**10.** Précurseur de denrées alimentaires ou de matières fourragères, pouvant être obtenu en utilisant une composition selon une des revendications 1 à 8.

**11.** Amidon résistant à base d'α-1,4-D-glucanes linéaires insolubles dans l'eau comme médicament, dans lesquels linéaire signifie que le degré de ramification en position 2-, 3-, ou 6- ne dépasse pas la valeur de 0,5 %.

**12.** Amidon résistant selon la revendication 11, dans lequel le médicament est un agent gastro-intestinal.

**13.** Composition pharmaceutique ou de médecine vétérinaire, comprenant une teneur en amidon résistant à base d'α-1,4-D-glucanes linéaires insolubles dans l'eau, dans lesquels linéaire signifie que le degré de ramification en position 2-, 3-, ou 6- ne dépasse pas la valeur de 0,5 %.

**14.** Composition selon la revendication 13, comprenant en outre un autre additif fonctionnel.

**15.** Composition selon la revendication 14, dans laquelle l'additif fonctionnel est un additif de denrées alimentaires ou de matières fourragères, en particulier un probiotique, par exemple un bifidobacterium.

**16.** Composition selon la revendication 15 , dans laquelle l'additif fonctionnel est une substance médicinale active, en particulier un médicament curatif.

**17.** Utilisation d'amidon résistant à base d'α-1,4-D-glucanes linéaires insolubles dans l'eau pour la préparation d'un médicament pour le traitement et/ou la prophylaxie de maladies gastro-intestinales, dans lesquels linéaire signifie que le degré de ramification en position 2-, 3-, ou 6- ne dépasse pas la valeur de 0,5 %.